(19) **Europäisches Patentamt**

**European Patent Office**

**Office européen des brevets**

(11) **EP 0 998 931 A1**

(12) **EUROPEAN PATENT APPLICATION**
published in accordance with Art. 158(3) EPC

(43) Date of publication:
**10.05.2000 Bulletin 2000/19**

(51) Int. Cl.[7]: **A61K 31/59**

(21) Application number: **98919555.7**

(86) International application number:
**PCT/JP98/02080**

(22) Date of filing: **12.05.1998**

(87) International publication number:
**WO 98/51313 (19.11.1998 Gazette 1998/46)**

(84) Designated Contracting States:
**AT BE CH CY DE DK ES FI FR GB GR IE IT LI LU MC NL PT SE**

(30) Priority: **16.05.1997 JP 12675997**

(71) Applicant:
**SANTEN PHARMACEUTICAL CO., LTD.
Higashiyodogawa-ku, Osaka-shi, Osaka 533-8651 (JP)**

(72) Inventors:
• **NAKATA, Katsuhiko
  Sakurai-shi Nara 633-0072 (JP)**
• **NAKAMURA, Masatsugu
  Nara-shi Nara 631-0074 (JP)**
• **HAZATO, Atsuo
  Hino-shi Tokyo 191-0062 (JP)**

(74) Representative:
**Peaucelle, Chantal
Cabinet Armengaud Aine
3, avenue Bugeaud
75116 Paris (FR)**

(54) **REMEDIES FOR DRY EYE**

(57) Purposes of the present invention are to find new effects and medical uses of $1\alpha$, 24-dihydroxyvitamin $D_3$, and the present invention provides 1) therapeutic agents for dry eye, 2) inhibitors of keratinocyte differentiation in corneal epithelial cells and 3) inhibitors of type I transglutaminase (TG-I) expression, each containing $1\alpha$, 24-dihydroxyvitamin $D_3$ as an active ingredient.

EP 0 998 931 A1

Printed by Xerox (UK) Business Services
2.16.7 (HRS)/3.6

**Description**

Technical Field

The present invention relates to therapeutic agents for dry eye containing $1\alpha$, 24-dihydroxyvitamin $D_3$ as an active ingredient.

Background Art

Dry eye is a disease group which indicates symptoms of eye drying and causes a corneal epithelial disorder owing to abnormalities of tear fluid. Examples of symptoms of dry eye are symptom of eye drying, conjunctival injection, itching, a sense of a foreign body, etc. When dry eye becomes serious, it sometimes causes visual loss. Recently, dry eye is said also to cause eyestrain.

Causes of incidence of dry eye are various, and exemplified by a decrease of tear secretion, a decrease of viscous components and excess of oily components in a tear fluid, a qualitative change in secretions, but many causes have not been elucidated yet.

Instillation of artificial tears has been carried out since old times with the view of compensating a tear fluid as symptomatic treatment of dry eye. Various drugs have been also studied with the aim of healing a keratocojunctival epithelial disorder, which is a main disorder in dry eye.

It is reported that keratinized cells due to abnormal differentiation exist in corneal epithelial cells of patients with dry eye (Clinical Study of Allergy, 12, 59-61 (1992)). The differentiation of corneal epithelial cells into keratinized cells leads to loss of normal function which the cells originally have. Keratinized cells do not exist in normal human corneal epithelial cells, and keratinized cells are considered to participate in incidence of dry eye.

Recently, it was reported that type I transglutaminase (TG-I) is expressed in human corneal epithelial cells (Invest. Ophthalmol. Vis. Sci., 37, S355 (1996)). TG-I is an enzyme which is involved in keratinization of keratinocytes, and the expression of TG-I is considered to be an index of keratinocyte differentiation. Accordingly, if the expression of TG-I can be inhibited in corneal epithelial cells of patients with dry eye, it is expected that keratinocyte differentiation is inhibited in corneal epithelial cells, which leads to therapy of dry eye.

$1\alpha$, 24-Dihydroxyvitamin $D_3$, which is an active ingredient in the present invention, is a so-called activated vitamin $D_3$ and it is a known compound described in Examined Japanese Patent Publication No. 9222/1978. The compound is known to have medical uses such as therapeutic agents for renal failure and rickets (Examined Japanese Patent Publication Nos. 31150/1978 and 31151/1978), therapeutic agents for diseases in which thromboxane $A_2$ participates such as ischemic heart disease, cerebrovascular disease and bronchial asthma (Japanese Patent No. 2543627), therapeutic agents for chronic hepatitis (Published Japanese translation of PCT No. 504249/ 1991), anti-inflammatory antipyretic analgesic (Examined Japanese Patent Publication No. 19207/1991).

In addition, WO96/29079 discloses ophthalmic application of a compound group which is comprehensively called activated vitamin D. This publication states that activated vitamin D is useful for preventing optical transparency inhibition, ocular hypertension or refraction error caused by cell hyperplasia and/or cellular metabolite hyperplasia in anterior ophthalmic cells in the process of postoperative repair of tissues damaged by an ophthalmological operation. However, the publication does not disclose application of activated vitamin D to dry eye at all.

Though $1\alpha$, 24-dihydroxyvitamin $D_3$, is a compound which is useful for various medical uses as mentioned above, it is a very interesting subject to find new pharmaceutical effects and medical uses thereof.

Disclosure of the Invention

Studying precisely on new pharmaceutical effects of $1\alpha$, 24-dihydroxyvitamin $D_3$, the present inventors found that $1\alpha$, 24-dihydroxyvitamin $D_3$ inhibits TG-I expression in corneal epithelial cells. Namely, $1\alpha$, 24-dihydroxyvitamin $D_3$, was found to inhibit keratinocyte differentiation in corneal epithelial cells and to be useful as a therapeutic agent for dry eye.

The present invention relates to 1) therapeutic agents for dry eye, 2) inhibitors of keratinocyte differentiation in corneal epithelial cells, and 3) inhibitors of type I transglutaminase (TG-I) expression which contain $1\alpha$, 24-dihydroxyvitamin $D_3$ as an active ingredient. Effects thereof will be described in detail in pharmacological test below.

Stereoisomers are present in $1\alpha$, 24-dihydroxyvitamin $D_3$, which is the active ingredient of the present invention, and all of them are included in the present invention. In particular, $1\alpha$, 24 (R)-dihydroxyvitamin $D_3$ is preferably used among them.

$1\alpha$, 24-Dihydroxyvitamin $D_3$, which is the active ingredient of the present invention, can be administered orally or parenterally. Examples of dosage forms are tablets, capsules, granules, powders, injections, ophthalmic solutions, eye ointments, etc., and ophthalmic solutions are particularly preferable. They can be prepared by the conventional methods. For example, ophthalmic solutions can be prepared, if necessary, using isotonic agents such as sodium chloride and concentrated glycerin; buffers such as sodium phosphate and sodium acetate; surfactants such as polyoxyethylene sorbitan monooleate (hereinafter referred to as "polysorbate 80"), stearic polyoxyl 40 and polyoxyethylene hydrogenated castor oil; stabilizers such as sodium citrate and sodium edetate; preservatives such as benzalkonium chloride and paraben. pH of the ophthalmic solutions can be in a range which is acceptable to ophthalmic preparations. A preferred pH range is 4 to 8. Oral preparations such as tablets, capsules, granules and powders can be produced by adding optionally volume increasing agents such as lactose, crystalline cellulose, starch and vegetable oil; lubricants such as magnesium stearate and talc; binders such as hydroxypropylcellulose and polyvinyl pyrrolidone; disintegrators such as calcium carboxymethylcellulose and low-substituted hydroxypropylmethylcellulose; coating agents such as hydroxypropylmethylcellulose, macrogol and silicone resin; or film coating agent such as gelatin.

The dosage can suitably be selected depending on symptoms, age, dosage form, and the like. In the case of ophthalmic solutions, the dosage is one to several times instillation per day of 0.00001 to 1% (w/v), preferably 0.0001 to 0.1% (w/v) solution. In the case of oral preparations, the usual daily dosage is 0.1 to 5000 mg, preferably 1 to 100 mg, which can be given in a single dose or a few divided doses.

Formulations and results of pharmacological test are shown below, and they are intended for better understanding the present invention but are not to limit the scope of the present invention.

Best Mode for Carrying out the Invention

Formulations

Typical formulations using $1\alpha$, 24-dihydroxyvitamin $D_3$ as an active ingredient are shown below.

1) Ophthalmic solution (in 100 ml)

| $1\alpha$, 24 (R)-Dihydroxyvitamin $D_3$ | 1 mg |
|---|---|
| Sodium chloride | 900 mg |
| Polysorbate 80 | 100 mg |
| Sterile purified water | q.s. |

Ophthalmic solutions having $1\alpha$, 24 (R)-dihydroxyvitamin $D_3$ concentrations of, for example, 0.0001% (w/v), 0.0003% (w/v), 0.003% (w/v), 0.01% (w/v), 0.03% (w/v) and 0.1% (w/v) can be prepared by changing amounts of $1\alpha$, 24 (R)-dihydroxyvitamin $D_3$ and additives appropriately.

2) Eye ointment (in 100 g)

| $1\alpha$, 24 (R)-Dihydroxyvitamin $D_3$ | 1 g |
|---|---|
| Liquid paraffin | 20 g |
| White vaseline | 74 g |
| Purified lanolin | 5 g |

3) Tablet (in 100 mg)

| $1\alpha$, 24 (R)-Dihydroxyvitamin $D_3$ | 10 mg |
|---|---|
| Lactose | 63 mg |
| Cornstarch | 15 mg |
| Calcium carboxymethylcellulose | 5 mg |
| Hydroxypropylcellulose | 5 mg |
| Magnesium stearate | 1 mg |
| Talc | 1 mg |

The tablets prepared by the above-mentioned formulation can be coated with a coating agent (for example, an ordinary coating agent such as hydroxypropylmethyl cellulose, macrogol or silicone resin) to obtain coated tablets.

Pharmacological Test

TG-I, which is enzyme participating in proliferation of keratinocytes, is expressed in human corneal epithelial cells (Invest. Ophthalmol. Vis. Sci., 37, S355 (1996)), and TG-I is considered to be an index indicating differentiation of keratinocytes. Existence of keratinized cells due to abnormal differentiation is observed in corneal epithelial cells of patients with dry eye (Clinical Study of Allergy, 12, 59-61 (1992)). If the expression of TG-I can be inhibited in corneal epithelial cells, it is expected that keratinocyte differentiation is inhibited in corneal epithelial cells, which leads to treatment of dry eye.

Accordingly, the present inventors examined effects of $1\alpha$, 24-dihydroxyvitamin $D_3$ on expression of TG-I in corneal epithelial cells.

Specifically, expression amounts of TG-I mRNA were measured by the RT-PCR method according to the method of Toshino et al. (Invest. Ophthalmol. Vis. Sci., 37, S355 (1996)) using cultured SV40 immortalized human corneal epithelial cells (Invest. Ophthalmol. Vis. Sci., 36, 614-621 (1995)). Glyceraldehyde-3-phosphoric acid dehydrogenase (G3PDH) was used as an internal standard in consideration of scatter of template total RNA amounts.

A comparative study was done using 1α, 25-dihydroxyvitamin $D_3$, which is one of activated vitamin $D_3$ compounds having chemical structure similar to that of 1α, 24-dihydroxyvitamin $D_3$, as a comparative compound.

Experimental method

Cultured SV40 immortalized human corneal epithelial cells ($1 \times 10^6$ cells) were cultured in a culture medium (DMEM/F12) containing a test compound under a condition of 37°C and 5% $CO_2$ for 72 hours. After culturing, total RNA was extracted according to the AGPC method. Then, RT-PCR was carried out using a primer which is specific for TG-I m RNA or G3PDH m RNA using a Takara RNA-PCR kit (AMV). PCR products were electrophoresed on 1% agarose gel, and bands were detected with a transilluminator. Cells cultured in a culture medium containing no test compound in the same manner were used as a control.

The expression amounts of TG-I m RNA were calculated according to the following equation 1 so that an expression amount in the control was 100.

$$\text{Expression amount} = (B/A) \times 100 \quad \text{Equation 1}$$

A: A band area of a TG-I amplified fragment on the basis of a band area of a G3PDH amplified fragment in the control

B: A band area of a TG-I amplified fragment on the basis of a band area of a G3PDH amplified fragment in the presence of the test compound

Results

Table 1 shows results in cases where the cells were cultured in a culture medium containing 1α, 24 (R)-dihydroxyvitamin $D_3$ at a concentration of 10 nM, 100 nM and 1000 nM, respectively. Table 1 also shows a result in case where the cells were cultured in a culture medium containing 1α, 25-dihydroxyvitamin $D_3$, which was the comparative compound, at a concentration of 1000 nM.

Table 1

|  | Expression amount |
| --- | --- |
| Control | 100 |
| 1α, 24 (R)-Dihydroxyvitamin $D_3$ |  |
| (10 nM) | 66 |
| (100 nM) | 39 |
| (1000 nM) | 22 |
| 1α, 25-Dihydroxyvitamin $D_3$ |  |
| (1000 nM) | 48 |

As shown in Table 1, in the case of 1α, 24 (R)-dihydroxyvitamin $D_3$, the expression amounts of TG-I m RNA in the corneal epithelial cells were 66% (34% inhibition) at a concentration of 10 nM, 39% (61% inhibition) at a concentration of 100 nM and 22% (78% inhibition) at a concentration of 1000 nM. Thus, concentration-dependent inhibition was observed. On the other hand, in the case of 1α, 25-dihydroxyvitamin $D_3$, which was the comparative compound, the expression amount was 48% (52% inhibition). Even though the concentration was 1000 nM, the exhibited inhibitory effect was only one tenth or less as much as that of 1α, 24 (R)-dihydroxyvitamin $D_3$.

As apparent from the above-mentioned results of the pharmacological test, 1α, 24-dihydroxyvitamin $D_3$ has an inhibitory effect on differentiation of keratinocytes in corneal epithelial cells by inhibiting the expression of TG-I in corneal epithelial cells, and it is useful as a therapeutic agent for dry eye.

Industrial Applicability

The present invention provides 1) therapeutic agents for dry eye, 2) inhibitors of keratinocyte differentiation in corneal epithelial cells and 3) inhibitors of type I transglutaminase (TG-I) expression, each containing 1α, 24-dihydroxyvitamin $D_3$ as an active ingredient, as new medical uses of 1α, 24-dihydroxyvitamin $D_3$.

**Claims**

1. A therapeutic agent for dry eye containing 1α, 24-dihydroxyvitamin $D_3$ as an active ingredient.

2. An inhibitor of keratinocyte differentiation in corneal epithelial cells containing 1α, 24-dihydroxyvitamin $D_3$ as an active ingredient.

3. An inhibitor of type I transglutaminase expression containing 1α, 24-dihydroxyvitamin $D_3$ as an active ingredient.

# INTERNATIONAL SEARCH REPORT

| International application No. |
|---|
| PCT/JP98/02080 |

| A. CLASSIFICATION OF SUBJECT MATTER |
|---|
| Int.Cl⁶ A61K31/59 |

According to International Patent Classification (IPC) or to both national classification and IPC

| B. FIELDS SEARCHED |
|---|
| Minimum documentation searched (classification system followed by classification symbols)<br>Int.Cl⁶ A61K31/59 |
| Documentation searched other than minimum documentation to the extent that such documents are included in the fields searched |
| Electronic data base consulted during the international search (name of data base and, where practicable, search terms used)<br>CA (STN) |

| C. DOCUMENTS CONSIDERED TO BE RELEVANT |||
|---|---|---|
| Category* | Citation of document, with indication, where appropriate, of the relevant passages | Relevant to claim No. |
| PX | WO, 97/18817, A1 (Kiyoshi Kita),<br>May 29, 1997 (29. 05. 97),<br>Particularly Claims & AU, 9653476, A | 1, 2 |
| X | WO, 96/29079, A1 (Seiji Itoh),<br>September 26, 1996 (26. 09. 96),<br>Particularly Claims ; page 6, line 25 to page 7,<br>line 7 ; page 8, line 26 to page 10, line 10<br>& EP, 815862, A1 & AU, 9649559, A | 2 |
| A | H. Sato et al., "Tacalcitol(1,24(OH)₂D₃, TV-02)<br>inhibits phorbolester-induced epidermal<br>proliferation and cutaneous inflammation, and<br>induces epidermal differentiation in mice", Vol. 288,<br>Arch Dermatol Res, 1996, p.656-663 | 3 |
| A | Takashi Matsunaga et al., "Effects of 1,24(R)-(OH)₂D₃<br>on epidermal cell differentiation in mice (in<br>Japanese)", Vol. 21, No. 12, Basic Pharmacology &<br>Therapeutics (Jpn Pharmacol Ther), 1993, p.4575-4579 | 3 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search<br>July 14, 1998 (14. 07. 98) | Date of mailing of the international search report<br>July 28, 1998 (28. 07. 98) |
|---|---|
| Name and mailing address of the ISA/<br>    Japanese Patent Office | Authorized officer |
| Facsimile No. | Telephone No. |

Form PCT/ISA/210 (second sheet) (July 1992)